Europäisches Patentamt

European Patent Office

Office européen des brevets

(19)

(11) Publication number: **0 117 060**

A2

# (12) EUROPEAN PATENT APPLICATION

(21) Application number: 84300300.5

(22) Date of filing: 18.01.84

(51) Int. Cl.³: **C 12 N 15/00**
C 07 H 21/04, C 12 N 5/00
//C12R1/91, C12N9/72,
C12N9/06, C12P21/02

(30) Priority: 19.01.83 US 459151

(43) Date of publication of application:
29.08.84 Bulletin 84/35

(84) Designated Contracting States:
AT BE CH DE FR GB IT LI LU NL SE

(71) Applicant: GENENTECH, INC.
460 Point San Bruno Boulevard
South San Francisco California 94080(US)

(72) Inventor: Levinson, Arthur David
3690 Ralston Avenue
Hillsborough California 94010(US)

(72) Inventor: Simonsen, Christian Clinton
1509 Parkview Avenue
San Jose California 95310(US)

(72) Inventor: Yelverton, Elizabeth McLeod
1624 Danromas Way
San Jose California 95129(US)

(74) Representative: Armitage, Ian Michael et al,
MEWBURN ELLIS & CO. 2/3 Cursitor Street
London EC4A 1BQ(GB)

(54) Methods of screening and amplification in eukaryotic host cells, and nucleotide sequences and expression vectors for use therein.

(57) A plasmid which comprises a DNA sequence coding for a DHFR protein with a low binding affinity for MTX. Also, plasmids which contain the foregoing sequence along with a sequence encoding a desired heterologous protein, each sequence operably linked to a sequence capable of effecting its expression.

Methods of transforming eukaryotic cells with the genetic sequences for a desired protein, selecting the successful transformants, and amplifying in the transformants, by employing the sequence coding for DHFR protein with low MTX binding affinity. Wild type eukaryotic cells may be used as hosts; it is not necessary to use mutants which have become deficient in a particular enzyme or cofactor.

./...

Croydon Printing Company Ltd.

Fig.5.

Docket 100/140

## METHODS OF SCREENING AND AMPLIFICATION
## IN EUKARYOTIC HOST CELLS, AND
## NUCLEOTIDE SEQUENCES AND EXPRESSION
## VECTORS FOR USE THEREIN

### Background of the Invention

The present invention relates to the field of transforming eukaryotic cells, selecting for transformants, and effecting increases in expression level of DNA sequences whose products are desired. In particular, the invention relates to employing a mutant gene which confers drug resistance on vertebrate cells to permit detection of cells which have been transformed with a vector, which vector also includes the coding sequence for a desired heterologous protein. The invention also relates to using the sequence conferring drug resistance to control the expression of the sequence for the desired protein product.

It has now become well known to utilize a microbial host for production of a heterologous protein. However, the use of, for example, bacterial strains as host cells, while conferring certain advantages with respect to ease of growth and harvest conditions, is frequently disadvantageous because of the lack of additional capabilities in such systems to modify, glycosylate, or otherwise manipulate the protein which results from the expression of a transforming sequence. For example, while bacteria may be successfully transfected and caused to express "thymosin α-1", the

polypeptide produced lacks the N-acetyl group characteristic of the "natural" thymosin α-1 found in the mammalian system. The production of tissue plasminogen activator, as disclosed in co-pending application (EPO Publication No. 0093619), while producing a true copy of the amino acid sequence of the mammalian protein, fails to glycosylate the peptide. Accordingly, in some instances it is desirable to utilize eukaryotic cells, such as, for example, transformed mammalian cells, or other eukaryotic cell lines, as host cells for the expression of the desired protein sequences.

Successful transformation of eukaryotic cell cultures and expression of sequences coding for desired protein in such cultures has been disclosed. See for example, U.S. Application Serial No. 298,236 filed August 31, 1981 (EPO Publication No. 0073657); and U.S.A. Application Serial No. 326,980 filed Dec. 3, 1981 (EPO Publication No. 0073656), both incorporated herein by reference.

It is desirable in carrying out transformations pursuant to heterologous protein production to have a suitable method for identification of transformed hosts to permit easy selection of the desired transformants. In bacteria, for example, genes whose products confer resistance to ampicillin or tetracycline are commonly used as markers. However, such standard techniques for vertebrate host cells are not as commonly available. Recently, it has been found that by using cells which are deficient in β-isopropyl-malic acid dehydrogenase as host cultures, the DNA sequence coding for this enzyme can be used as a marker. The transformed host cells will grow on a medium deficient in the end product of, but containing the substrate for, this enzyme. (EPO Patent Application No.81-110085.8 (EP 54223).) Other mutant cell lines which are deficient in various enzymes such as AMP pyrophosphate phosphoribosyltransferase (aprt⁻), and thymidine kinase (tk⁻). (Wigler, et al., Proc. Natl. Acad. Sci. (USA) 76:1373, 1979) and a Chinese hamster ovary (CHO) cell mutant deficient in dihydrofolate

reductase (DHFR) activity (dhfr⁻) (Urlab, Gail and Chasin, Lawrence, Proc. Natl. Acad. Sci. (USA) 77:4216, 1980) are also available. These mutant cell lines may conveniently be used as host cells, permitting selection of transformants when the transforming DNA includes an expressable sequence encoding the enzymes in which the cells are deficient, simply by growing the transformed cells on minimal medium i.e., deficient in the products of the enzymes encoded by the endogenously missing genes, and selecting those colonies capable of growth.

Dominant marker systems are not unknown. For example neomycin resistance is conferred by a sequence whose presence is detected by using G418 as a selecting agent (Southern, P.J. and Berg, P.; J. Mol. Appl. Genet. 1:127, 1982).

The present invention permits the use of "wild type" vertebrate host cells by utilizing a DNA sequence which encodes a protein conferring drug resistance on the mammalian or other vertebrate cell line. This capability is particularly useful in conjunction with an expression vector which includes the coding sequence for a heterologous protein desired to be produced by the host cells. In a preferred embodiment of the present invention, the susceptibility of eukaryotic cells to methotrexate (MTX) a known inhibitor of DHFR is utilized.

It has long been recognized that certain cells are capable of overcoming MTX inhibition due to a number of mechanisms, including the ability to decrease the uptake of MTX (Sirotnak, F.M. et al., Cancer Res. 28:75, 1968; Fischer, G.A., Biochem. Pharmacol. 11:1233, 1962); by increasing the production of DHFR (Biedler, J.L. et al. Cancer Res. 32:153, 1972; Chang, S.E. et al., Cell 7:391, 1976; and by manufacture of DHFR which has less affinity for MTX (Flintoff, W.F., Somat. Cell Genet. 2:245, 1976); Wigler, et al. Proc. Natl. Acad. Sci. (USA) 77:3567, 1980). Thus, a low affinity DHFR has been detected in a Chinese hamster ovary (CHO) cell line mutant, A29 which permitted the growth of such cells in the presence of 20 µg/ml of MTX, a concentration that would kill all cells containing only normal levels of a wild type DHFR. DNA isolated from A29 cells has been ligated to the bacterial plasmid pBR322 and used to transform NIH 3T3 mouse fibroblast cells, and the resulting transformed cells were capable of surviving high concentrations of MTX. A29 genomic DNA has also been used in conjunction with a plasmid containing the genome of hepatitis B virus (HBV) to transform NIH 3T3 cells which cells, upon selection with MTX, gave rise to colonies expressing HBV proteins. (Christman, J.K. et al., Proc. Natl. Acad. Sci. (USA) 79:1815, 1982).)

It is also known that in cells which are MTX resistant due to high levels of DHFR, the DHFR gene is amplified. (Schimke, R.T. et al.,.Science 202:1051, 1978). Wigler, (supra) also found that the DNA sequences in pBR322 were amplified in cells transformed using A29 DNA ligated into pBR322 when the cells were grown in increased concentrations of MTX. In any event cotransfected sequences can be amplified by selecting with MTX.

The generation of dihydrofolate reductases with altered inhibitor affinity has been observed by several groups. (Albrecht, et al. Cancer Res. 32:1539, 1972; Flintoff, D.F. supra; Jackson, R.C. et al., Eur. J. Cancer 13:567, 1967; Goldie, J.H. et al., Eur. J. Cancer 16:1539, 1980).

The present invention is directed to a specifically modified DHFR coding sequence which results in DHFR protein conferring MTX resistance apparently due both to decreased MTX affinity and to amplification of its gene, thus enabling growth of wild type cells transformed with sequences coding for the modified DHFR at MTX concentrations which would ordinarily be lethal. It also comprises, in another aspect, utilizing such elevated concentrations of MTX to amplify not only the DHFR gene itself, but a coding sequence for a desired heterologous peptide with which the cells have been cotransformed.

## Summary of the Invention

In one aspect, the invention herein relates to a nucleotide sequence which codes for the amino acid sequence of DHFR 3T6 R400, a DHFR enzyme produced by a mutant line of mouse fibroblast cells.

In a second aspect, the invention is directed to a plasmid which comprises a DNA sequence coding for a DHFR protein with a low binding affinity for MTX. In another aspect, the invention is directed to plasmids which contain the foregoing sequence along with a sequence encoding a desired heterologous protein, each sequence operably linked to a sequence capable of effecting its expression. Thus, the invention includes not only plasmids which are capable of expressing the gene for suitably modified DHFR, but also plasmids which co-express the gene for another desired protein.

In still another aspect, the invention relates to methods of transforming eukaryotic cells with the genetic sequences for a desired protein, selecting the successful transformants, and amplifying in the transformants, by employing the sequence coding for DHFR protein with low MTX binding affinty. In the process of this invention, wild type eukaryotic cells may be used as hosts; it is not necessary to use mutants which have become deficient in a particular enzyme or cofactor.

The invention is further directed to a method for selecting vertebrate cells by utilizing an expression vector conferring drug resistance. The invention also includes the host cell lines transformed according to the methods, and with the vectors, described hereinabove.

Finally, the invention also includes desired heterologous protein produced by the methods claimed.

Brief Description of the Drawings

Figure 1a shows a restriction map of the R400 cDNA. Figure 1b sets forth the nucleotide and deduced amino acid sequence of DHFR 3T6 R400.

Figure 2 shows a comparison of mutant and wild type DHFR cDNA.

Figure 3 shows the construction of DHFR expression plasmids, pFR400 and pFD11.

Figure 4 shows the inhibition of growth by MTX of cells which are transformed with pFD11 and pFR400. (pFD11 carries wild type DHFR; pFR400 carries DHFR 3T6 R400.)

Figure 5 shows the derivation of plasmid pCVSVEHBVE400R400 (pEHER), a plasmid expressing mutant DHFR and HBsAg.

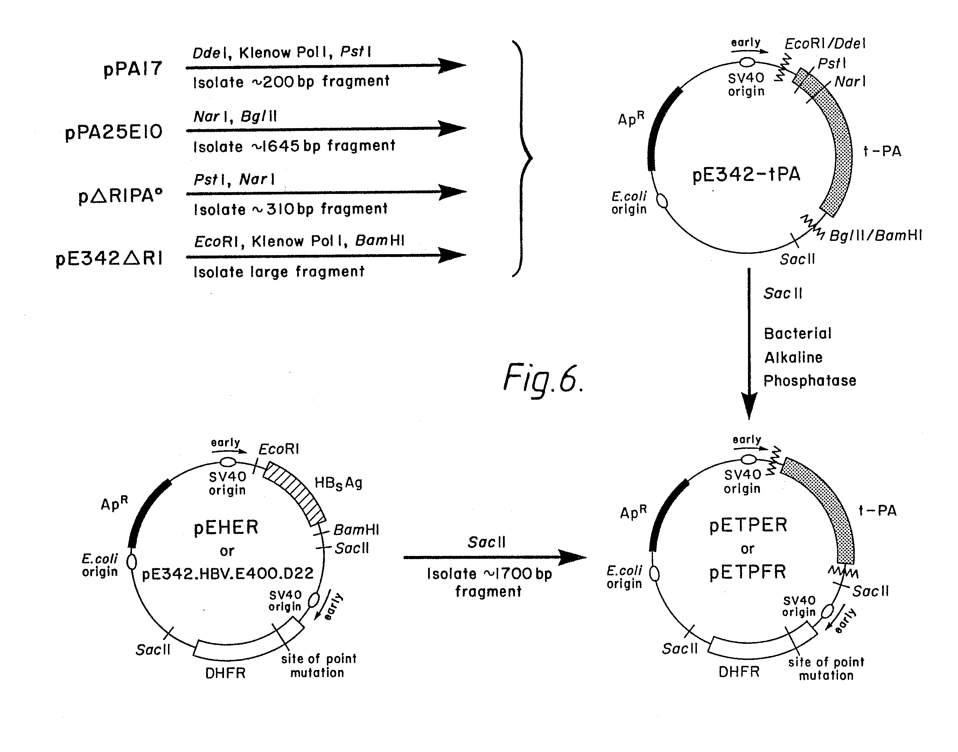

Figure 6 shows the derivation of plasmid pETPA.ER400 (pETPER), a plasmid expressing mutant DHFR and tPA.

Detailed Description

A.    Definitions

As used herein, "nucleotide sequence" refers to a nucleic acid which is comprised of a series of nucleotides in a 5' to 3' phosphate diester linkage which may be either an RNA or a DNA sequence. If a DNA, the nucleotide sequence may be either single or double stranded. Similarly, "DNA sequence" refers to both single and double stranded embodiments.

0242L

When the formula for a nucleotide sequence is given, it will be given starting at the 5' end with the left and terminating with the 3' end at the right. If the sequence is intended to represent a double stranded DNA, it will be understood that a complementary strand runs with the 3' at the left and the 5' at the right.

"DHFR protein with a low binding affinity for MTX" has, herein, a functional definition. This is a DHFR protein which, when generated as the DHFR-type protein within cells, will permit the growth of MTX sensitive cells in a medium containing 0.2 µg/ml or more of MTX. It is recognized that such a functional definition depends on the facility with which the organism produces the "DHFR protein with a low binding affinity for MTX", as well as upon the protein itself. However, as used in the context of this invention, such a balance between these two mechanisms should not be troublesome. The invention operates with respect to conferring the capability of surviving these levels of MTX, and it is not consequential whether the ability to do so is impacted by increased expression in addition to the innate nature of the DHFR produced.

"MTX sensitive cells" are cells which, unless genetically altered, or otherwise supplemented, will fail to grow under ambient and medium conditions suitable for the cell type when the MTX concentration is 0.2 µg/ml or more.

"DHFR 3T6 R400" refers to the altered DHFR found in the 3T6 line of mouse fibroblasts as described by Haber, D.A. et al., J. Biol. Chem. 256:9501 (1981); Haber, D.A. and Schimke, R.T., Cell 26:355 (1981), both incorporated herein by reference.

"Desired heterologous protein" refers to a protein which is desired to be expressed in a host cell, but which the host cell normally does not itself produce, at least in large enough quantities to be detectable, and which is not normally necessary for the cells continued existence. For example, viral coat antigen for

0242L

hepatitis B virus (HBsAg) is heterologous for mammalian cells not infected by virus. Production of HBsAg in host recombinant cells is, however, desirable as a source of vaccine.

"Expression vector" includes vectors which are capable of expressing DNA sequences contained therein, where such sequences are operably linked to other sequences capable of effecting their expression. It is implied, although not always explicitly stated, that these expression vectors must be replicable in the host organisms either as episomes or as an integral part of the chromosomal DNA. Clearly a lack of replicability would render them effectively inoperable. In sum "expression vector" is given a functional definition, and any DNA sequence which is capable of effecting expression of a specified contained DNA code is included in this term as it is applied to the specified sequence.

B.    Host Cell Cultures and Promoters

The invention is operable or useful as appropriate in host cell cultures which are normally sensitive to MTX (or in vertebrate cell cultures sensitive to other suitable drug). The host cells in the examples herein are therefore not intended to be limiting, but representative. Any cell which is prevented from growth by MTX levels 0.2 µg/ml or less will be workable.

A number of vertebrate lines have been grown in tissue culture in recent years. (Tissue Culture, Acad. Press, (1973)). Among the most commonly used as host cells for the production of heterologous proteins are the COS-7 line of monkey fibroblasts (Gluzman, Cell 23:275, 1981), and continuous cell lines such as Chinese hamster ovary cells (CHO), HeLa cell lines, L cells, and Vero cells.

For use in mammalian cells, the control functions on the expression vectors are generally provided by viral material. For

0242L

example, commonly used promoters are derived from polyoma, Adenovirus 2, and most frequently Simian Virus 40 (SV40). The early and late promoters of SV40 virus are particularly useful because both are obtained easily from the virus as a fragment which also contains the SV40 viral origin of replication (Fiers, et al., Nature 273:113, 1978) incorporated by reference. In the presence of the SV40 early proteins (denoted T and/or t antigens), expression vectors containing this origin fragment will replicate in permissive cell nuclei and will promote the transcription of sequences lying 3' of the early and late promoters at high levels (Gluzman, et al., Cold Spring Harbor Symposium Quant. Biol. 44:293, 1980). Gluzman, supra.)

Smaller or larger SV40 fragments may also be used, provided there is included the approximately 250 bp sequence extending from the HindIII site toward the BglI site located in the viral origin of replication.

The example which is set forth hereinbelow describes use of CHO, and L cells as host cells, and expression vectors which include the SV40 origin of replication as a promoter. However, it would be well within the skill of the art to use analogous techniques to construct expression vectors for expression of desired protein sequences in alternative eukaryotic host cell cultures.

C.    Detailed Description of Preferred Embodiment

As described in detail hereinbelow, cDNA has been prepared from mouse fibroblast 3T6-400 cells, which cells are mutants capable of growing in relatively high levels of MTX. Other DHFR mutants could similarly be used as source of mRNA for construction of appropriate cDNA. The scope of the invention includes such other alternatives, as well as the specific DHFR embodiment employed in the example. Further, modified DNA sequences capable of encoding "DHFR protein with a low binding affinity for MTX" can be prepared

0242L

synthetically according to the method of Goeddel, et al., as described in U.S. Patent No. 4,342,832.

The cDNA obtained as described herein is ligated into an expression plasmid derived from pBR322, containing the SV40 origin oriented such that early promoter directs transcription of the DHFR sequence, a polyadenylation signal, a plasmid origin of replication, and an ampicillin resistance marker. Construction of this plasmid is described below. Clearly, however, other expression vectors, employing control mechanisms derived from other eukaryotic organisms, could also be used. Similarly, in addition to the sequences encoding hepatitis B surface antigen (HBsAg) and tissue plasminogen activator (tPA), as described in detail below, expression vectors capable of expressing heterologous genes such as those for interferons, growth hormones, and the like may be created by inserting the appropriate coding sequence.

In short, the example set forth below serves, not to limit the invention, but merely to illustrate it.

C.1    Isolation of mRNA

Mutant 3T6 R400 cells were obtained, with permission, from Dr. R. Schimke (Haber and Schimke) (supra). Cultures of 3T6 R400 were grown, in Dulbeccos modified Eagles medium, obtained from Grand Island Biologicals, comprising simple salts, nutrients and supplemented with calf serum, to a cell density of approximately $10^7$ cells/150 mm tissue culture dish. Cell monolayers from 3 plates were washed with sterile phosphate buffered saline (PBS) and lysed by the addition of approximately 5 ml per $10^7$ cells of a solution containing 0.65 percent Triton X-100, 0.01 M Tris, pH8, 0.15 M NaCl, and 0.015 M $MgCl_2$. After 5-10 minutes at 4°C, the mixture was centrifuged at 1500 x g to remove cell debris, and the supernatant supplemented with EDTA, pH 8, to a concentraton of 0.025 M, NaCl to 0.4 M, and sodium dodecyl sulfate (SDS) to 0.1

0242L

percent. The solution was then extracted with two volumes buffered phenol, centrifuged to separate the phases, and re-extracted with two volumes of a 1:1 mixture of phenol and chloroform. Two volumes of ethanol were added to the aqueous phase, the mixture chilled at -20°C for over 6 hours. RNA was recovered by centrifugation at 16000 x g at 4°C for 30 minutes. The pellet was washed with ethanol, dried, and resuspended in water. About 4 mg of RNA was obtained.

Approximately 2 mg of this RNA was taken up in about 2 ml of a buffer composed of 0.01 mTris pH 7.5, 0.4M NaCl, and .01 M EDTA. 0.1 percent SDS was added and the solution loaded onto an oligo dT-cellulose column preequilibrated with the same buffer. The solution was recycled through the column twice and the column washed with 20-30 ml of the above buffer. mRNA was eluted by the addition of 0.01M Tris pH 7.5, 0.01 M EDTA. The fractions containing mRNA were concentrated by ethanol precipitation and resuspended in sterile water. Approximately 50 µg mRNA was obtained.

C.2    Preparation of cDNA:

The procedure is as described in Goeddel D. et al., Nucleic Acids Research 8:4057 (1980), incorporated herein by reference, and was applied with results subsequently to be described.

Briefly, for 10 µg mRNA, the initial $^{32}$P labeled single strand DNA was formed by placing the mRNA prepared as described above in 100 µl of reverse transcriptase buffer solution (20 mM, Tris, pH 8.3, 20 mM KCl, 8 mM MgCl$_2$, 30 mM beta mercaptoethanol (BMSH)) which is 1 mM each in the four dNTPs, 100 microcuries in dCTP, and contains approximately 2 µg oligo-dT (12-18) (Collaborative Res.) primer, and 20 units of reverse transcriptase (BRL). The solution was incubated at 42°C for 30 minutes; then quenched in ice water and boiled for 3 minutes. After cooling, denatured protein was removed by centrifugation, and the supernatant recovered.

The complementary DNA chain was then formed by adding an equal volume of 1/2 times reverse transcriptase buffer containing 10 units DNA polymerase I (Klenow) (NEN). The solution was incubated at 14°C for 3 hours and then at 4° for 15 hours. Reaction was stopped and protein extracted by vortexing with 100 μl of phenol saturated with buffer (5 mM Tris, pH 8, 200 mM NaCl, 1 mM EDTA) and adding 100 μl chloroform. The aqueous phase was then removed and the nucleic acid precipitated with about 2-2.5 times the volume of ethanol at -20°C. The precipitate was spun down and washed with cold 70 percent ethanol. (The foregoing method of protein removal and nucleic acid recovery was followed throughout.)

The pellet was then dissolved in 100 μl S1 buffer (25 mM NaOAc, pH 4.5, 1 mM $ZnCl_2$, 300 mM NaCl) and treated with approximately 80 units SI nuclease (BRL) at 37°C for 90 minutes to cleave the "hairpin" in the cDNA.

EDTA was added to 0.025 M and the mixture phenol-choloform extracted as set forth above. Nucleic acids were precipitated using 3 volumes ethanol at dry ice temperatures.

The pellet was resuspended in 45 μl water and 5 μl 50 percent glycerol, 0.1 percent xylene cyanol (XC), 0.1 percent bromophenol blue (BPB) added. The solution was loaded onto 6 percent acrylamide gel prepared as described by Goeddel (supra) (5 ml 10x TEB, 0.5 ml 10 percent ammonium persulfate (APS), 7.5 ml 40 percent acrylamide, 36 ml water, 50 μl TEMED.) After 60 minutes at 200 volts the gel was stained in ethidium bromide to determine size locations. (Acrylamide gel electrophoresis was run for varying times in the foregoing manner throughout.) The band corresponding to the desired size was cut, and counted (Cerenkov), using a scintillation counter and electroeluted into about 400 μl 1/10 TPE buffer and recounted to

0242L

determine yield of cDNA. To this was added 50 μl 3 M NaOAc, pH 7.2 and 1000 μl cooled ethanol. This cDNA was stored at -20°C.

In the specific preparation set forth 10 μg mRNA, prepared as described, were employed in the reverse transcriptase reaction using oligo dT as primer, and the band corresponding to fragments of greater than 700 bp was recovered from the acrylamide gel for use in preparation of clones.

## C.3    Preparation of Cloned DHFR 3T6-R400 cDNA

### C.3.a  Transfection and Growth:

100 ng (as judged by recovered radioactivity) of the cDNA as prepared above was tailed with poly C and inserted into pBR322 as follows:

To 100 ng cDNA, suspended in 10 μl water, were added 10 μl of 10x cacodylate buffer, 10 μl 10 mM dCTP, 68 μl water, 1 μl 100 mM $CoCl_2$, and 10 units terminal transferase. The mixture was incubated at 37°C for 3 minutes and protein removed by phenol-chloroform extraction as described above. To the supernatant was added 0.8 ng PstI cleaved pBR322 tailed with poly G. The mixture was extracted first with phenol and then with chloroform, and to the aqueous phase was added 3 μl 3 M NaOAc, pH 7, and 100 μl ethanol. The spun down (nucleic acid) precipitate was resuspended in 90 μl water plus 10 μl 10x annealing buffer (100 mM Tris, pH 7.5, 2.5 mM EDTA, 1 M NaCl) and incubated at 70°C diminishing to 37°C overnight, and diminishing to 4° during the next day.

50 μl of the annealed vector and cDNA thus prepared were then used to transform 200 μl of E. coli K12 strain 294 cells, (ATCC 31446) by calcium phosphate precipitation as originally described by Mandel, A. and Higa, J. Mol. Biol. 53:154 (1970), and adapted by Goeddel (supra) incorporated herein by reference. After 1 hour, the cells were heat shocked at 42°C for 70 seconds and held for 90 minutes in 5 ml LB at 37°C. 600 µl aliquots of cell culture were then plated onto 8 large plates of LB containing 5 µg/ml tetracycline (Tc), and the transformed colonies were recovered.

The colonies were picked into fresh plates, incubated overnight, and transferred onto nitrocellulose filters (Schleicher a Schuell BA85) placed on LB plates containing 5 µg per ml Tc. The remaining portions of the colonies in the dishes were preserved by storing at -20°C.

The transferred portions of the colonies were incubated for 8 to 9 hours at 37°C, and then amplified by transferring from the filters to plates containing 12.5 µg/ml chloramphenicol and incubating at 37°C overnight.

## C.3.b  Probe Preparation

Nick-translated probe was prepared by the method of Davis et al., Advanced Bacterial Genetics, Cold Spring Harbor Laboratory, Cold Spring Harbor, New York (1980) incorporated herein by reference. A mixture of DNA fragments was obtained by digesting with PstI and BglII the cDNA insert from murine DHFR cDNA coding region as described by Nunberg, J.H. et al., Cell 19:355 (1980), incorporated herein by reference, 300 ng of the 700 bp Pst-BglII fragment was treated in 30 µl Pol 1 buffer (50 mM Tris pH 7.5, 10 mM $MgSO_4$, 1 mM dithiothreitol, 250 uM dG,A,TTP, 50 µg/ml BSA) to which was added 50 µCi alpha $^{32}$P-dCTP (Amersham No. 10205) and 0.5 µl of a 1/40,000 dilution (in Pol 1 buffer) of 5 mg/ml DNase. After 1 minute at 20°, the mixture was chilled to 4°, and 1 µl of DNA polymerase I (holoenzyme) was added and the mixture incubated 3 hours at 14°C. Labeled probe was separated from unincorporated nucleotides with a Sephadex G-50 column.

0242L

C.3.c  Selection and Sequencing of a Clone Encoding 3T6 R400

Each nitrocellulose filter containing transformed, cultured and amplified colonies was probed for the desired cDNA sequence as follows:

Each filter was laid successively on paper towels saturated with a lysis solution (0.5 M NaOH, 1.5 M NaCl) for 3 minutes, with a neutralizing solution (0.5 M Tris, 3 M NaCl pH 7.5) for 15 minutes and with 2x standard sodium citrate solution for 15 minutes and then dried.  The filters were baked in vacuo at 80°C for 60 minutes.

To the filter was added a solution of 50 percent formamide, 5 x SSPE (1 x SSPE = 0.18 M NaCl, 10 mM (Na 1.5) $PO_4$, 1 mM $Na_2$ EDTA, pH7.0.), 0.3 percent SDS, and 100 µg/ml salmon sperm DNA (denatured by the addition of 1/10 volume 1.5 M NaOH followed by 1/10 volume 1.5 M HCl).  The filter was incubated for 2 hours at 42°, and the solution then replaced with the hybridization solution which was made 10 percent (w/v) with sodium dextran sulfate.  A solution containing salmon sperm DNA and $10^6$ cpm of probe were denatured and added to the filter.  After 16 hours at 42°, the filters are washed 5 times with 200 mls of 2x SSPE, .3 percent SDS at 42°, dried, and placed against x-ray film to locate colonies containing $^{32}P$, i.e. those which hybridize with probe.

In carrying out the foregoing procedure, 2000 colonies which contained DNA inserts greater than 600 base pairs were screened.

These 2000 colonies on nitrocellulose filters were hybridized with probe obtained by nick translation as described above.  Twelve of the colonies hybridized strongly, and the plasmid DNA from them was isolated for further analysis.

The procedure for this small scale plasmid isolation was described by Birnboim, H.C. and Doly, J., Nucleic Acids Res. 7:1513

0242L

(1979). The isolated plasmids were analyzed by digestion with TaqI followed by electrophoresis on polyacrylamide gels. The logic behind such analysis is that the DHFR coding sequence in normal murine DHFR has two TaqI restriction sites located 190 base pairs apart and situated at the 5' end of the cDNA. Accordingly, the desired full-length cDNA sequences should contain this 190 base pair segment. Five of the plasmids showed this band. In general, large scale plasmid preparations were purified from these identified cultures using the cleared lysate method of Clewell, D.B. and Helinski, Biochemistry 9:4428 (1970), incorporated herein by reference and purified by column chromatography on Biorad A-50 Agarose.

From among these five, the cloned DNA with the longest 5' untranslated region was designated pR400-12. This sequence extended over 100 base pairs 5' of the TaqI site adjacent to the initiation codon, and over 300 base pairs 3' of the termination codon, as judged by restriction analysis using TaqI, HinfI, HaeIII, HpaII and PstI.

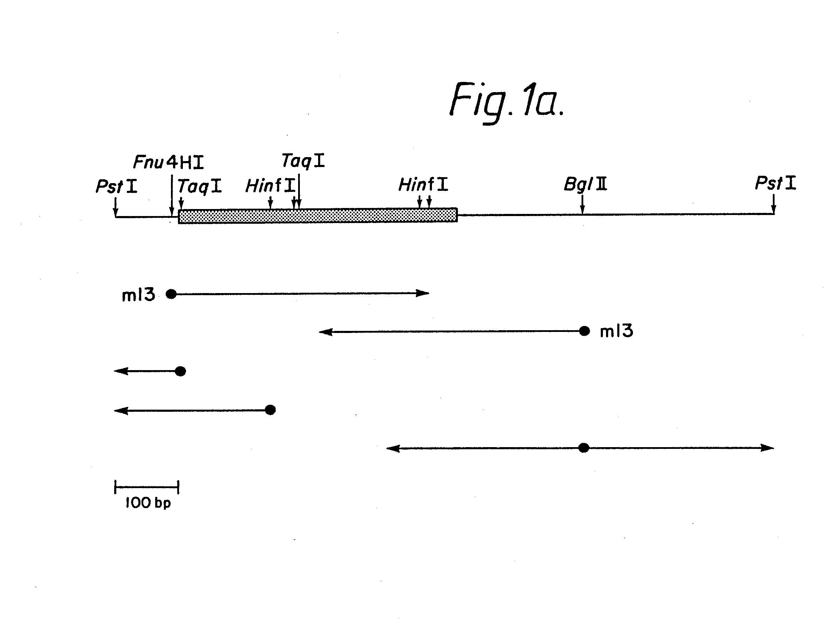

The cDNA insert of this plasmid was sequenced end to end using the methods of Maxam, A and Gilbert, W., Methods of Enzymology 65:499 (1980) a combination of the method of Chen, et al., Nature 299:529 (1982) with that of Smith, A.J.H., Methods of Enzymology 65:565 (1980), a di-deoxy chain termination method. Figure 1a illustrates this sequencing strategy.

Fragments of the cloned cDNA containing plasmids were prepared for sequencing by treating 20 µg of plasmids with 20 units of the appropriate restriction enzyme or sequence of restriction enzymes in approximately 200 µl solution containing the appropriate buffer for the restriction used (or sequence of buffers); each enzyme incubation was at 37°C for one hour. After incubation with the enzyme, protein was removed and nucleic acids recovered by phenol-cloroform extraction and ethanol precipitation as set forth

0242L

above. The nucleic acids were then resuspended in 40 µl of loading buffer for loading onto 6 percent polyacrylamide gel as described above for sizing.

In application of the Maxam and Gilbert Method (supra), pR400-12 DNA was digested with either HinfI, TaqI, or BglII, dephosphorylated using calf intestinal alkaline phosphatase, and phenol-chloroform extracted. The 5' termini were labeled with $^{32}$P-ATP in the presence of 1 µl T4 polynucleotide kinase (NEN, 18 u/µl) for 30' at 37°.

The labeled DNAs were again phenol chloroform extracted and ethanol precipitated. The HinfI and TaqI DNAs were resuspended in 1x gel buffer, and electrophoresed on an acrylamide gel. The HinfI 1800 bp and TaqI 800 bp bands were cut out, electroeluted, and phenol-chloroformed and ethanol precipitated using the standard method. These fragments were digested as follows:

a) TaqI 800 digested with PvuI.
b) HinfI 1800 digested with EcoRI.
c) BglII (whole plasmid) digested with PstI.

These digestion mixtures were electrophoresed on an acrylamide gel and the bands cut out which correspond to:

a) TaqI 500 bp fragment containing 5' end of cDNA labeled at TaqI site and spanning PstI site of pBR322.

b) HinfI 1500 bp fragment labeled at the 5' most HinfI site and spanning the PstI site of pBR322.

c) BglII 300 bp and 700 bp fragments labeled at BglII site and ending at the PstI site where they join pBR322 in pR40012.

0242L

These fragments were then sequenced.

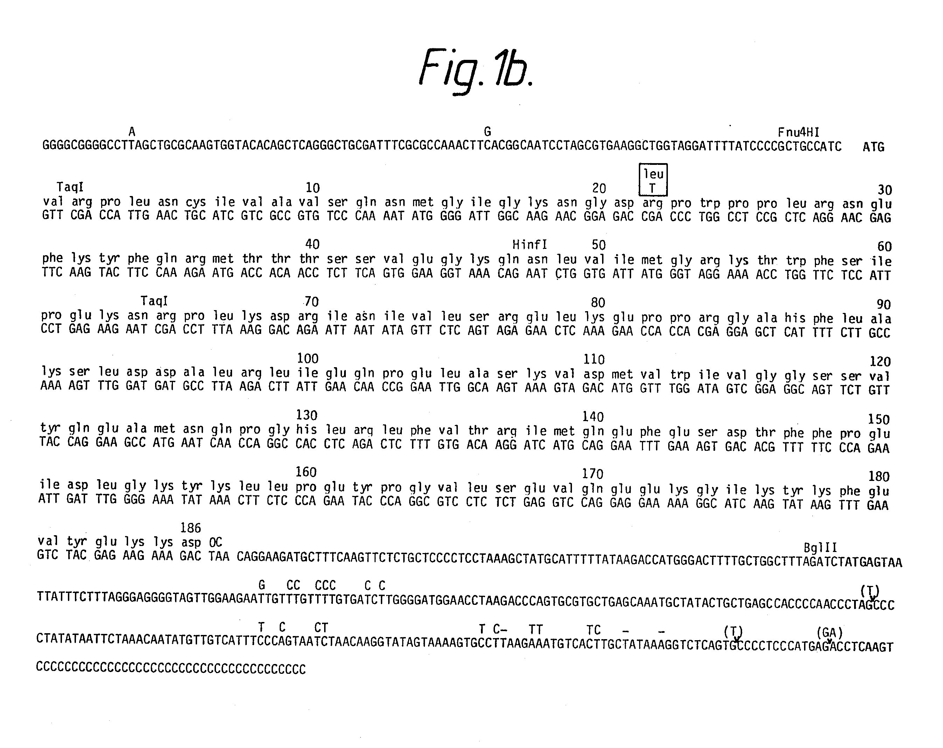

For M13 vector sequencing, the DHFR insert was digested with Fnu4HI, made blunt with Klenow DNA polymerase I, and subcut with BglII. The 660 bp fragment generated by such treatment was isolated from an acrylamide gel, phenol and chloroform extracted, and ethanol precipitated as described above. Approximately 0.2 ug of this fragment was ligated into SmaI-BglII digested M13 mp8 and 9 as described by Chen et al., supra in combination with Smith, et al., (supra). Figure 1 shows the complete sequence of the cDNA insert.

Comparison of the sequence analysis of the R400 and normal DHFR sequence shows only one base change in the coding region of the altered enzyme when compared to the wild type. (An "alteration" at position +397 is due to a sequencing error in the original report (Nunberg et al. supra) The alteration at position 68 represents the substitution of a guanine residue for thymine, thus substituting an arginine for a leucine in the peptide. Figure 1 gives the amino acid sequence of the 3T6 R400 enzyme.

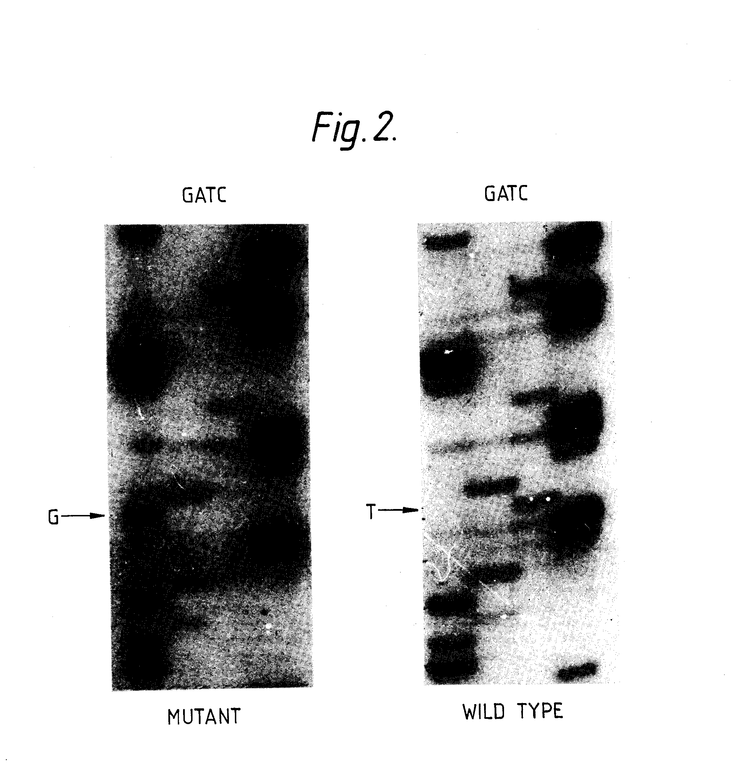

In order to verify this sequence difference, normal cDNA isolated from pDHFR-11 was sequenced coincidentally with cDNA from pR400.12. Figure 2 shows that the normal cDNA has a band corresponding to a T residue at the same position occupied by a G residue in the altered enzyme cDNA.

C.4    Construction of DHFR-3T6 R400 Expression Vectors

In general, expression plasmids for 3T6 R400 were assembled by ligating approximately equimolar amounts of the desired components, which components were suitably end tailored to provide correct matching, with T4 DNA ligase. Approximately 10 units ligase were required for 0.5 ug quantities of vector and sequence components. The resulting plasmid vectors were then cloned by transforming E. coli K12 strain 294 (ATCC 31446). The

0242L

transformation and cloning, and selection of transformed colonies were carried out as described above. Presence of the desired sequence was confirmed by isolation of the plasmids from selected colonies, restriction analysis and/or DNA sequencing as described above.

Expression plasmids were constructed by cleaving the R400-12 plasmid with the restriction enzyme Fnu 4HI. The ends were filled in with Klenow DNA polymerase I as described above. These were then subcut with BglII and electrophoresed on polyacrylamide gel.

The 660 base pair band encompassing the DHFR coding region was thus isolated and inserted into pCVESVHBV (described below) which contained an SV40 origin, a polyadenylation signal from hepatitis B virus, a plasmid origin of replication, and an ampicillin resistance marker.

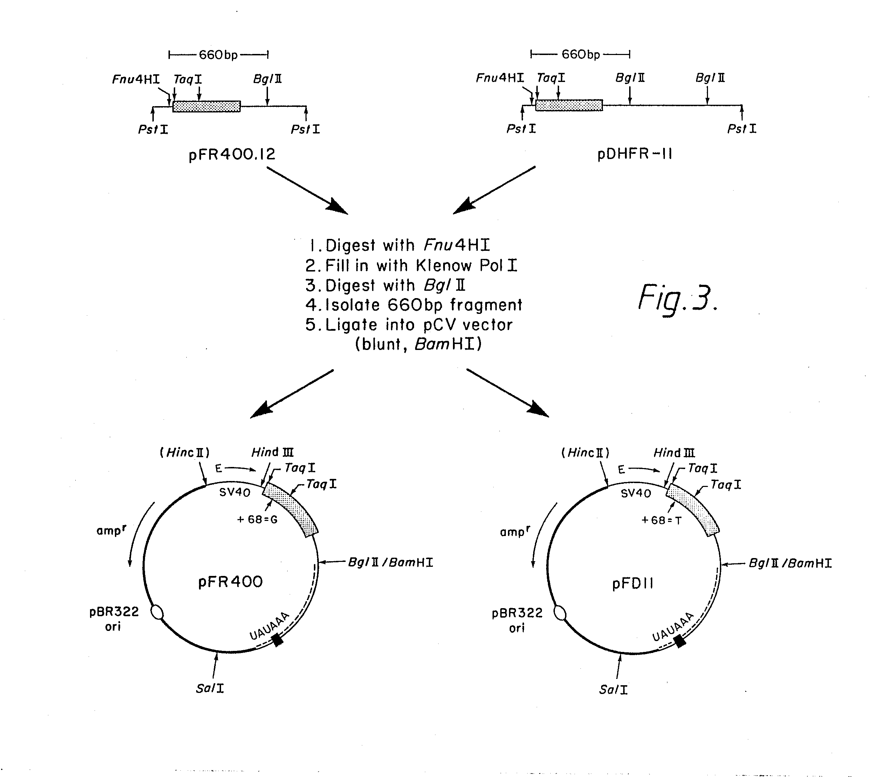

This vector plasmid was obtained as follows: The 540 bp Hind II-HindIII fragment encompassing the SV40 origin of replication (Liu et al., DNA 1:213, 1982) was ligated into plasmid pML (Lusky, M. and Botchan, M., Nature 293:79, 1981) between the EcoRI site and the HindIII site. The plasmid EcoRI site and SV40 HindII site were made blunt by the addition of Klenow DNA polymerase I in the presence of the 4 dNTPs prior to digestion with HindIII. The resulting plasmid, pESV was digested with HindIII and BamHI and the 2900 bp vector fragment isolated. To this fragment was ligated a HindIII-BglII fragment of 2025 bp from HBV modified to contain a polylinker (DNA fragment containing multiple restriction sites) at the EcoRI site. The HBV fragment encompasses the surface antigen gene and is derived by EcoRI-BglII digestion of cloned HBV DNA described by Liu et al. supra (DNA 1:213, 1982). The double stranded linker DNA fragment (5'dAAGCTTATCGATTCTAGAATTC3'...) was digested with HindIII and Eco RI and added to the HBV fragment, converting the EcoRI-BglII fragment to a HindIII-BglII fragment. Although this could be done as a 3 part ligation consisting of linker, HBV fragment, and vector, it is more convenient and was so performed to first add the HindIII-EcoRI linker to the cloned HBV DNA and then excise the HindIII-BglII fragment by codigestion of the plasmid with those enzymes. The resulting plasmid, pCVESVHBV, contains a bacterial origin of replication from the pBR322 derived pML, and ampicillin resistance marker, also from pML, an SV40 fragment oriented such that the early promoter will direct the transcription of the ingested HBV fragment, and the surface antigen gene from HBV. The HBV fragment also provides a polyadenylation signal for the production of polyadenylated mRNAs such as are normally formed in the cytoplasm of mammalian cells. The HBsAg coding region is removed by digesting with EcoRI, filling-in the ends with Klenow DNA polymerase as previously described, and subcutting with the BamHI. Into this area is inserted the Fnu 4HI-BglII fragment from the R400 DHFR cDNA. The resulting plasmids are diagrammed in Figure 3. pFD11 was constructed using the Fnu4HI-BglII fragment of wildtype DHFR cDNA plasmid pDHFR-11 (Nunberg, et al., supra). pFR400 was constructed using the similar fragment from pR400.12.

## C.5    Transfection of Host Cells with pFR400

Tissue culture cells were transfected by mixing 1 μg of the DHFR expression plasmid as prepared above with 10 μg rat carrier DNA in a volume of 250 μl, 0.25 M $CaCl_2$, followed by dropwise addition of 250 μl HEPES buffered saline (280 mM NaCl, 1.5 mM $Na_2PO_4$, 50 mM HEPES, pH 7.1). After 30 minutes at room temperature, the solution was added to tissue culture cells growing in 60 mm plastic tissue culture dishes. CHO 1, CHO DHFR-DUX-B11, and Ltk⁻cells were used. The dishes contained 3 ml culture medium appropriate to the host cell.

For CHO 1 and CHO DHFR-DUX-B11 cells, the medium was Ham F-12 media (Gibco) supplemented with 10 percent calf serum, 100 μ/ml penicillin 100 μg/ml streptomycin, and 2 μmM L-glutamine. For

Ltk⁻cell line, the medium used was Dulbecco modified Eagle's medium (DMEM) supplemented as above.

After 3-16 hours, the medium was removed and the cells were washed with 20 percent glycerol in phosphate buffered saline. Fresh medium was added to each plate and the cells were incubated for 2 more days.

Selection of transfected host cells was carried out by trypsinizing the cells after 2 days growth (which comprises treating the cells with sterile trypsin 0.5 mg/ml containing 0.2 mg/ml EDTA) and adding ~ $3 \times 10^5$ cells to 10 mm tissue culture plates with selective media. For dhfr⁻ cells the medium is a formulation of (F-12 GIBCO) medium lacking glycine, hypoxanthine, and thymidine (GHT⁻ medium). For DHFR⁺ host cells, MTX is added to the normal growth medium. Colonies arising from cells which take up and express the DHFR plasmid were apparent within 1-2 weeks.

Controls were run using transfection conditions with no plasmid and with plasmid pFD-11 containing normal DHFR.

Table 1 shows the frequency of colony formation using dhfr⁻ CHO cells when the colonies are selected using GHT medium. The colonies were plated onto 10 cm dishes and fed with Hams medium F-12 lacking glycine, hypoxanthine, and thymidine, supplemented with 7 percent dialyzed calf serum. In some cases MTX at the indicated concentration was added. Cells were re-fed 3 days later with the same medium in each case. Colonies were counted 10 days after transfection by staining with crystal violet.

Table 1

Frequency of Colony Formation by DHFR⁻ Cells Transfected
with Mutant and Wildtype DHFR Expression Plasmids.

| MTX (nM) | Transfectional Efficiency Colonies/$\mu$g/$10^6$ Cells | | |
|---|---|---|---|
| | No Plasmid | pFD11 | pFR400 |
| 0 | <0.3 | >$10^3$ | 330 |
| 1 | -- | 330 | 310 |
| 5 | <0.3 | 260 | 230 |
| 10 | -- | 140 | 240 |
| 25 | — | 30 | 250 |
| 50 | <0.3 | <0.3 | 240 |
| 100 | <0.3 | <0.3 | 200 |
| 250 | -- | -- | 170 |
| 500 | -- | -- | 85 |
| 1000 | -- | -- | 75 |
| $10^4$ | -- | -- | 45 |
| $10^5$ | -- | -- | 10 |

Note: "--" refers to "not tested."

As seen in Table 1, the plasmid containing the normal DHFR
was highly successful in transforming the colonies in the absence of
MTX as was the pFR-400 plasmid, albeit slightly less efficient. At
higher concentrations of MTX, at which the cells transformed by
normal DHFR are completely incapable of growth, transfectional
efficiency is maintained for the mutant, MTX-resistant DHFR. As
seen, concentrations greater than $10^4$ nM (45 $\mu$g/ml of MTX) were
capable of providing a suitable selective medium for these
transfected cells.

0242L

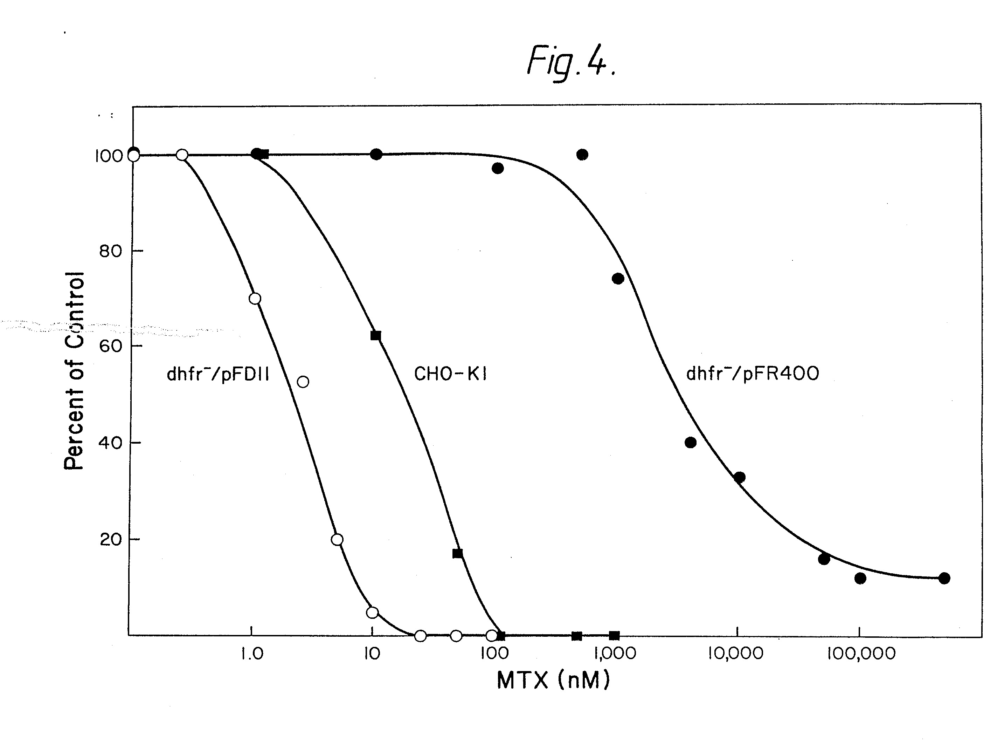

A deficiency of the wild type DHFR as a selectable marker in tissue culture systems is the relatively low degree of MTX resistance conferred upon the transfected cells by the transfected wild type enzyme expression plasmid. This is illustrated in Figure 4 which shows the growth inhibition exerted by MTX on CHO-DUX-B11 (dhfr-) cells transfected with either pFR400 or pFD11 in comparison to wild type untransformed CHO-K1 cells. Individual colonies were isolated from pFR400 or pFD11 transfected dhfr- cells and propagated in the absence of MTX. Equal amounts of cells from two clones and of CHO-K1 cells were trypsinized and split into 60 mm dishes. Various levels of MTX were added to each dish, the cells incubated for 3 days, and then counted.

The pFD11 transfected cells were clearly less capable of growing in high levels of MTX than were the CHO-K1 (DHFR+) cells grown under identical conditions. The level of MTX which inhibits cell growth by 50 percent over the 3 day assay was approximately 3 nM for cells derived from the pFD11 transfected colony (pFD11.1) and was 20nM for CHO-K1 cells. In contrast to this, the colony (pFR400.1) isolated from the cells transfected with pFR400, was able to grow in extremely high levels of MTX. The 50 percent inhibition level for FR400.1 cells is approximately 3000 nM.

Table 2 shows the transfectional efficiency of pR400 in both DHFR+ and dhfr- cell lines. Transfection with pR400 results in the appearance of MTX resistant colonies at 100, 250, and 500 nM MTX. No colonies capable of being propagated arise from the cells transfected with the wild type enzyme expression plasmid pFD11 nor from cells transfected only with carrier DNA. This DHFR encoded by pFR400 is capable of being used as a dominant selectable marker in tissue culture cells by employing selection in MTX-supplemented medium.

0242L

Table 2

Frequency of Colony Formation by CHO-K1 and Ltk⁻
Cells Transfected with DHFR Expression Plasmids

| | | Transfectional Efficiency Colonies/$\mu$/$10^6$ Cells | | |
| MTX (nm) | Cell Line | No Plasmid | pFD11 | pFR400 |
|---|---|---|---|---|
| 250 | CHO | <1 | 5* | 270 |
| 500 | CHO | <1 | 5* | 90 |
| 100 | Ltk⁻ | <2 | <2 | 80 |
| 250 | Ltk⁻ | <2 | <2 | 44 |
| 500 | Ltk⁻ | <2 | <2 | 14 |

* Nonviable colonies which did not propagate upon subcloning.

C.6    Construction of an Expression Plasmid Containing the Sequence
for Hepatitis B Surface Antigen and 3T6 R400.

The components of the vector are obtained as follows:

The HBsAg protein coding sequence was prepared as follows: (Briefly, the origin of the Simian virus SV40 was isolated by digesting SV40 DNA with HindIII, and converting the HindIII ends to EcoRI ends by the addition of a converter (AGCTGAATTC). This DNA was cut with PvuII, and RI linkers added. Following digestion with EcoRI, the 348 base-pair fragment spanning the origin was isolated by polyacrylamide gel electrophoresis and electroelution, and cloned in pBR322. Expression plasmid pHBs348-E was constructed by cloning the 1986 base-pair fragment resulting from EcoRI and BglII digestion of HBV (Animal Virus Genetics, (Ch. 5) Acad. Press, N.Y., 1980) (which spans the gene encoding HBsAg) into the plasmid pML (Lusky et al., Nature 293:79, 1981) at the EcoRI and BamHI sites. (pML is a derivative of pBR322 which has deletion eliminating sequences which are inhibitory to plasmid replication in monkey cells). The resulting plasmid (pRI-Bgl) was then linearized with EcoRI, and the 348 base-pair fragment representing the SV40 origin region was

0242L

introduced into the EcoRI site of pRI-Bgl. The origin fragment can insert in either orientation. Since this fragment encodes both the early and late SV40 promoters in addition to the origin of replication, HBV genes could be expressed under the control of either promoter depending on this orientation (pHBS348-E representing HBs expressed under control of the early promoter). pE342 is modified by partially digesting with EcoRI, filling in the cleaved site using Klenow DNA polymerase I, and ligating the plasmid back together, thus removing the EcoRI site preceding the SV40 origin in pE342. The resulting plasmid, designated pE342ΔR1, is digested with EcoRI, filled in using Klenow DNA polymerase I, and subcut with BamHI. After electrophoresing on acrylamide gel, the approximately 3500 bp fragment is electroeluted, phenol-chloroform extracted, and ethanol precipitated as above.

The thus prepared p342E 3500 bp vector was digested with EcoRI and SacII and the 3500 bp fragment containing a portion of the HBsAg coding sequence, isolated from an acrylamide gel.

Plasmid pFR400 was digested with ClaI and SacII and the 720 bp fragment isolated.

The EcoRI-TaqI fragment spanning the HBV surface antigen gene was obtained by digesting HBV DNA cloned into pBR322 (Liu et al., DNA 1:213, 1982) with EcoRI and TaqI and isolating the approximately 2000 bp fragment on an acrylamide gel.

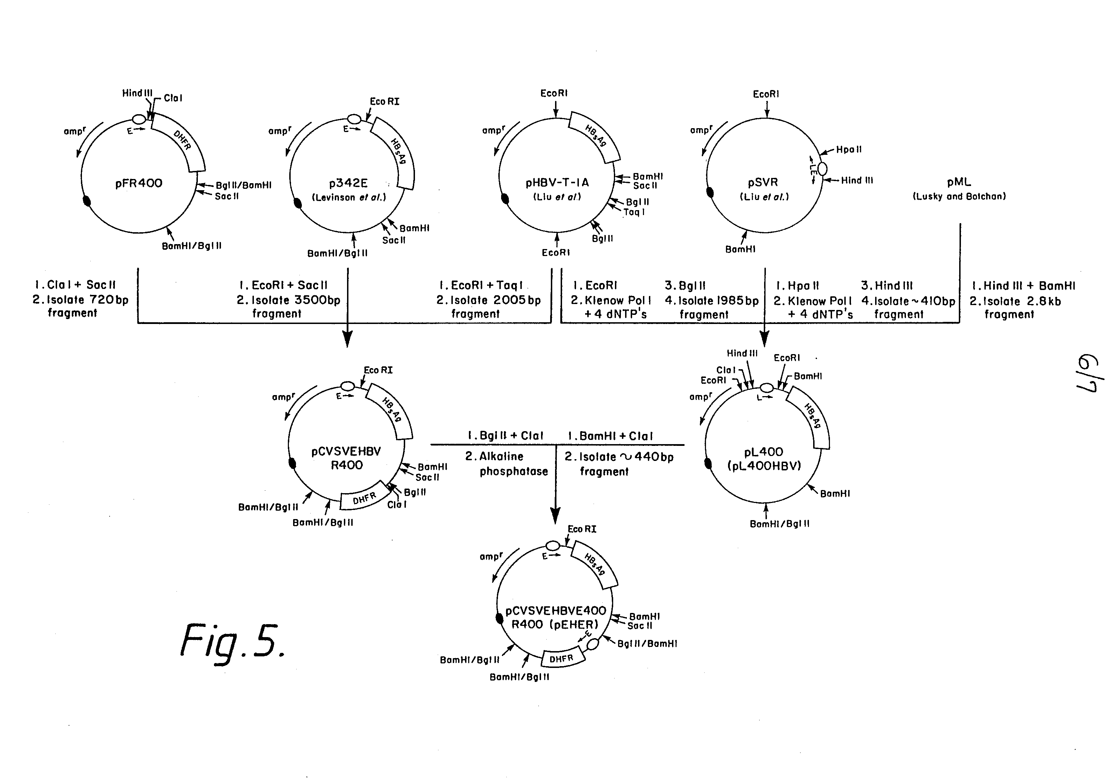

These three fragments were ligated together as shown in Figure 5, creating pCVSVEHBVR400.

An SV40 promoter was then inserted in front of the DHFR coding sequences using the following method. Plasmid pML was digested with HindIII and BamHI and the 3000 bp fragment isolated. Cloned SV40 viral DNA (Liu et al., supra) was digested with HpaII; ends were made blunt using Klenow DNA polymerase, and subcut with HindIII. The approximately 410 bp fragment encompassing the SV40 origin was isolated. Finally, cloned HBV DNA (Liu et al., supra) was digested with EcoRI, filled in using Klenow DNA polymerase, and subcut with BglII. The 1985 bp fragment was isolated on an acrylamide gel. The three fragments were ligated together as indicated in Figure 5, creating pL400.

The origin fragment of pL400 is flanked by a ClaI site on the early side of the origin and a BamHI site on the late side. pL400 was digested with ClaI and BamHI and the origin fragment was isolated on an acrylamide gel. Plasmid pCVSVEHBVR400 was digested with ClaI and BgIII treated with calf intestinal alkaline phosphatase (Boehringer). The origin fragment was added to the pCVSVEHBVR400 mixture and ligated, creating pCVSVEHBVE400R400 (also referred to herein as pEHER) as shown in Figure 5.

C.7    Expression of Heterologous Gene Sequences

Plasmid pEHER was used to transfect and select CHO-K1 DUX-B11 cells, CHO-K1, and Ltk⁻ cells as described above. Colonies are isolated by placing a glass cylinder around the colony and trypsinizing to detach the cells from the plate surface. The cells are placed into culture dishes-with the appropriate media as set forth above, propagated for several days, and then assayed for the production of HBsAg using a radio-immunoassay kit (Abbot) as described by Liu et al. (supra).

As shown in Table 3, surface antigen is produced in extremely high levels in colonies isolated from pEHER transfected cell lines. Similar levels of HBsAg production are observed from dhfr- cells transfected with the analogous wild type DHFR/HBsAg plasmid (pE342.HBV.E400.D22), but no colonies arise from Ltk- or CHO cells transfected with this plasmid and thus no surface antigen is produced. When cells are transfected with a tissue plasminogen activator expressing plasmid (see below), colonies also produce the cotransfected gene encoded protein.

Table 3

| Cell Line | Plasmid | MTX (nM) | Viable Colonies/ug/ $10^6$ Cells | Colonies Expressing | |
|-----------|---------|----------|------------------|-------|-----|
| | | | | HBsAg | TPA |
| DHFR⁻ | FR400 | 0 | 330 | – | – |
| DHFR⁻ | FDII | 0 | 1000 | – | – |
| DHFR⁻ | EHER | 0 | 300 | 6/6 | – |
| DHFR⁻ | ETPER | 0 | 300 | – | 12/12 |
| CHOk1 | FR400 | 250 | 270 | – | – |
| CHOk1 | FDII | 250 | <2 | – | – |
| CHOk1 | EHER | 250 | 270 | 3/4 | – |
| CHOk1 | EHER | 500 | 75 | 4/4 | – |
| CHOk1 | EHER | 1000 | 40 | 6/6 | – |
| CHOk1 | ETPER | 250 | 250 | – | 2/4 |
| CHOk1 | ETPER | 500 | 70 | – | 4/4 |
| CHOk1 | ETPER | 1000 | 40 | – | 4/4 |
| Ltk⁻ | FR400 | 250 | 45 | – | – |
| Ltk⁻ | FDII | 250 | <2 | – | – |
| Ltk⁻ | EHER | 100 | 100 | 6/6 | – |
| Ltk⁻ | EHER | 250 | 45 | 6/6 | – |

Indicated cell lines were transfected with the appropriate plasmid as described above, and were split at 2 days post-transfection into the level of Mtx in selective media as described before. Colonies were subcloned using cloning rings and were passaged in selective media. HBsAg expression was monitored using the Ausria$^{(tm)}$ RIA kit by Abbott as performed by Liu _et al_., (_supra_). Tissue plasminogen activator was encoded by pE342TPAER400 (pETPER) and assayed using the fibrin overlay technique described herein.

## C.8 Amplification of Expression of tPA

As a cotransfected gene, the cDNA insert from a plasmid containing the sequence encoding human tissue plasminogen activator (tPA) is inserted into a pFR400 expression plasmid as follows:
0242L

cDNA plasmids encoding tPA have been described by Goeddel et al., Patent Application Serial No. 374,860, filed May 5, 1982 (EPO Publication No. 0093619), which is hereby incorporated by reference. Human melanoma cells (Bowes) were cultured to confluent monolayers in 100 ml Earles Minimal Essential Media supplemented with sodium bicarbonate (0.12 percent final concentration), 2 mM glutamine and 10 percent heat-inactivated fetal calf serum. To confirm that the melanoma cells were actively producing human tissue plasminogen activator, human melanoma cells were cultured to confluency in a 24 well microtiter dish. Either in the presence or absence of 0.33 μM the protease inhibitor aprotinin, the cells were washed once with phosphate buffered saline and 0.3 ml of serum free methionine free medium was added. 75 μCi of $[^{35}S]$-methionine was added and the cells were labeled at 37° for 3 hours. At the end of the 3 hour labelling period the media was removed from the cells and treated with either tissue plasminogen activator specific IgG or pre-immune serum for immunoprecipitation (54). The immunoprecipitated products were displayed by electrophoresis on a 10 percent SDS-acrylamide gel. The slab gel was fixed, dried and subjected to fluorography.

Total RNA from melanoma cell cultures was extracted essentially as reported by Ward et al., J. Virol. 9:61 (1972). Cells were pelleted by centrifugation and then resuspended in 10 mM NaCl, 10 mM Tris-HCl pH 7.5, 1.5 mM $MgCl_2$. Cells were lysed by the addition of NP-40 (1 percent final concentration), and nuclei were pelleted by centrifugation. The supernatant contained the total RNA which was further purified by multiple phenol and chloroform extractions. The aqueous phase was made 0.2 M in NaCl and then total RNA was precipitated by the addition of two volumes of ethanol. Oligo-dT cellulose chromatography was utilized to purify mRNA from the total RNA preparations (Crea et al., Nucl. Acids Res. 8:2331, 1980). Typical yields from 10 grams of cultured melanoma cells were 5 to 10 milligrams of total RNA and 50-200 micrograms of Poly(A) plus mRNA.

0242L

Fractionation of PolyA$^+$ mRNA (200 µg) (Aviv et al., PNAS 69:1408, 1972) was performed by electrophoresis through urea-agarose gels. The slab agarose gel was composed of 1.75 percent agarose, 0.025 M sodium citrate, pH 3.8 and 6 M urea. Electrophoresis was performed for 7 hours at 25 milliamps and 4°C. The gel was then fractionated with a razor blade. The individual slices were melted at 70° and extracted twice with phenol and once with chloroform. Fractions were then ethanol precipitated and subsequently assayed by in vitro translation in a rabbit reticulocyte lysate system, Bethesda Research Lab, supplemented with dog pancreas microsomes as follows: Translations were performed using 25 µCi of [$^{35}$S] methionine and 500 nanograms of each gel slice RNA in a final volume of 30 µl containing 25 mM HEPES, 48.3 mM potassium chloride, 10 mM creatine phosphate. 19 amino acids at 50 mM each, 1.1 mM magnesium chloride 16.6 mM EDTA, 0.16 mM dithiothreitol 8.3 mM hemin, 16.6 µg/ml creatine kinase, 0.33 mM calcium chloride, 0.66 mM EGTA, 23.3 mM sodium chloride.

Incubations were carried out at 30°C for 90 minutes. Dog pancreas microsomal membranes prepared from rough microsomes using EDTA for removal of the ribosomes (Blobel et al., J. Cell Biol. 67:852, 1975) were treated with nuclease (Shields et al., J. Biol. Chem. 253:3753, 1978) and were present in the translation mixture at a final concentration of 7 A$_{260}$ units/ml. Translation products or immunoprecipitated translation products were analyzed by electrophoresis on 10 percent polyacrylamide gels in sodium dodecyl sulfate as previously described (Laemmli, Nature 227:680, 1970). The unstained slab gels were fixed, dried and subjected to fluorography (Bonner et al., Eur. J. Biochem. 46:83, 1974).

The resulting translation products from each gel fraction were immunoprecipitated with rabbit anti-human tissue plasminogen activator specific IgG. One major immunoprecipitated polypeptide band was observed in the translation of RNA fraction numbers 7 and 8 (migration of 21 to 24S) having a molecular weight of approximately 63,000 daltons. This band was not observed when preimmune IgG was used for immunoprecipitation which suggested these polypeptides were tissue plasminogen activator specific.

5 μg of gel fractionated mRNA (gel slice 7 mRNA) was used for the preparation of double stranded cDNA by standard procedures (52,65,66). The cDNA was size fractionated on a 6 percent polyacrylamide gel. The cDNA greater than 350 base pairs in length (125 ng) was electroeluted. 30 ng of cDNA was extended with deoxy(C) residues using terminal deoxynucleotidyl transferase and annealed with 300 ng of the plasmid pBR322 which had been similarly tailed with deoxy(G) residues at the PstI site (Chang et al., Nature 275:617, 1978). The annealed mixture was then transformed into E. coli K12 strain 294 (ATCC No. 31446). Approximately 4,600 transformants were obtained.

Purified human tissue plasminogen activator was obtained according to the procedure of disclosed references (EPO Publication No. 41776).

The molecule was scanned in order to locate regions best suited for making synthetic probes, as follows:

To make the proteins susceptible to digestion by trypsin it was reduced and carboxymethylated. A 2 mg sample of tissue plasminogen activator was first dialyzed against 0.01 percent Tween 80 over night at room temperature. The lyophilized protein was then dissolved in 12 ml of 0.56 M Tris-HCl buffer (pH 8.6), 8 molar in urea and 5 mM EDTA. The disulfide bonds were reduced by the addition of 0.1 ml of β-mercaptoethanol. This reaction was carried out under nitrogen for 2 hours at 45°C. The reduced disulfides were alkylated to the carboxymethyl derivative by the addition of 1.0 ml Of 1.4 M iodoacetic acid in 1N NaOH. After 20 minutes at room temperature the reaction was stopped by dialysis against 0.01 percent Tween 80 for 18 hours at room temperature and lyophilized.

0242L

The resulting lyophilized carboxymethylated protein was redissolved in 3 ml of 0.1 M sodium phosphate buffer (pH 7.5). Trypsin (TPCK) was added (1 to 50 ratio) and digested at 37°C. Aliquots (0.1 ml) were taken at 3 hours, 6 hours, and 12 hours. A second addition of trypsin was made at 12 hours. The reaction was stopped after 24 hours by freezing the sample until it could be injected on the HPLC. The progress of the digestion was determined by SDS gels on the aliquots. All gels were blank except for a faint band on the 3 hour aliquot. This indicated that the 24 hour digestion was complete and no large peptides remained.

A sample (ca. 0.5 ml) was injected into a high resolution Altex C-8 ultrasphere 5 μ column with two runs. A gradient of acetonitrile was made gradual (1 percent to 5 percent in 5 minutes, 5 percent to 35 percent in 100 minutes, 35-50 percent in 30 minutes). In one of the two preparative runs, the eluant was monitored at two wavelengths (210 nm and 280 nm). The ratio of the two wavelength absorptions was used to indicate the tryptophan containing peptides.

The peptide peaks most likely to contain tryptophan, or that were believed useful for other reasons, were sequenced first. This enabled the determination of the sequence around most of the tryptophans. After sequencing about 25 of the best possible peptide peaks, all the sequence data that could be aligned was pooled to obtain a preliminary model of the primary structure of tissue plasminogen activator. From this data and model, several possible probes were located.

The colonies were individually inoculated into wells of microtiter plates containing LB + 5 μg/ml tetracycline and stored at -20°C after addition of DMSO to 7 percent. Two copies of the colony library were grown up on nitrocellulose filters and the DNA from each colony fixed to the filter by the Grunstein Hogness procedure (PNAS 72:3961, 1975).

0242L

The $^{32}$P-labelled - TC($^A_G$)CA($^A_G$)TA($^C_T$)TCCCA probe was prepared (from the synthetic oligomer) (W-E-Y-C-D) 14-mer pool as described above. Filters containing 4,600 transformants were prehybridized for 2 hours at room temperature in 50 mm sodium phosphate pH 6.8, 5X SSC (Blin et al., Nucl. Acids Res. 3:2303, 1976), 150 µg/ml sonicated salmon sperm DNA, 5X Denhardt's solution 10 percent formamide and then hybridized with 50X $10^6$ counts per minute of the labelled probe in the same solution. After an overnight incubation at room temperature, the filters were washed 3 times at room temperature in 6X SSC, 0.1 percent SDS for 30 minutes, once in 2X SSC and then exposed to Kodak XR-5 x-ray film with Dupont Lightning Plus intensifying screens for 16 hours.

Plasmid DNA was isolated by a rapid method (Birnboim et al., Nucl. Acids Res. 7:1513, 1979) from all colonies showing a positive hybridization reaction. The cDNA inserts from these clones were then sequenced after subcloning fragments into the M13 vector mp 7 (Messing et al., Nucl. Acids Res. 9:309, 1981) and by the Maxam Gilbert chemical procedure (74).

The cDNA insert in clone 25E10 was demonstrated to be the DNA coding for tissue plasminogen activator by comparing its amino acid sequence with peptide sequence (See Supra) obtained from purified tissue plasminogen activator and by its expression product produced in E. coli as described in more detail infra. Clone 25E10 was 2304 base pairs in length with the longest open reading frame encoding a protein of 508 amino acids (MW of 56,756) and containing a 772 bp 3' untranslated region. This cDNA clone lacked the N-terminal coding sequences.

50 µg of pPA25E10 (supra) were digested with PstI and the 376 bp fragment isolated by electrophoresis on a 6 percent polyacrylamide gel. Approximately 3 µg of this fragment was isolated from the gel by electroeluting, digested with 30 units of

0242L

DdeI for 1 hour at 37° phenol and chloroform extracted, and ethanol precipitated. The resulting DdeI sticky ends were extended to blunt ends by adding 5 units of DNA polymerase I (Klenow fragment) and 0.1 mM each of dATP, dCTP, dGTP, dTTP to the reaction mixture and incubating at 4°C for 8 hours. After extraction with phenol and chloroform, the DNA was digested with 15 units of NarI for 2 hours and the reaction mixture electrophoresed on a 6 percent polyacrylamide gel. Approximately 0.5 µg of the desired 125 bp blunt end NarI fragment was recovered. This fragment codes for amino acids number 69 through 110 of the mature full length tissue plasminogen activator protein.

For isolation of the 1645 bp NarI-BglII fragment, 30 µg of pPA25E10 were digested with 30 units of NarI and 35 units of BglII for 2 hours at 37° and the reaction mixture electrophoresed on a 6 percent polyacrylamide gel. Approximately 6 µg of the desired 1645 bp NarI-BglII fragment were recovered.

The plasmid pdeltaRISRC is a derivative of the plasmid pSRCex16 (McGrath et al., Nature 295:423, 1982) in which the EcoRI sites proximal to the trp promoter and distal to the SRC gene have been removed by repair with DNA polymerase I, and the self-complementary oligodeoxynucleotide AATTATGAATTCAT (synthesized by the phosphotriester method was inserted into the remaining Eco RI sile immediately adjacent to the XbaI site. 20 µg of pdeltaRISRC were digested to completion with EcoRI, phenol and chloroform extracted, and ethanol precipitated. The plasmid was then digested with 100 units of nuclease S1 at 16°C for 30 minutes in 25 mM sodium acetate (pH 4.6), 1 mM ZnCl$_2$ and 0.3 M NaCl to create a blunt end with the sequence ATG. After phenol and chloroform extraction and ethanol precipitation, the DNA was digested with Bam H1, electrophoresed on a 6 percent polyacrylamide gel, and the large (4,300 bp) vector fragment recovered by electroelution.

0242L

The expression plasmid was assembled by ligating together 0.2 µg of vector, 0.06 µg of the 125 bp blunt end - NarI fragment and 0.6 µg of the 1645 bp NarI-BglII fragment with 10 units of $T_4$ DNA ligase for 7 hours at room temperature and used to transform E. coli strain 294 (ATCC No. 31446) to ampicillin resistance. Plasmid DNA was prepared from 26 of the colonies and digested with XbaI and EcoRI. Twelve of these plasmids contained the desired 415 bp XbaI-EcoRI and 472 bp Eco RI-fragments. DNA sequence analysis verified that several of these plasmids had an ATG initiation codon correctly placed at the start of amino acid number 69 (serine). One of these plasmids, pΔRIPA° was tested and produced the desired tissue plasminogen activator.

0.4 µg of the synthetic oligonucleotide 5' TTCTGAGCACAGGGCG 3' was used for priming 7.5 µg of gel fraction number 8 mRNA (supra) to prepare double stranded cDNA by standard procedures. The cDNA was size fractionated on a 6 percent polyacrylamide gel. A size fraction greater than 300 base pairs (36 ng) was electroeluted. 5 ng cDNA was extended with deoxy(C) residues using terminal deoxycytidyl transferase and annealed with 50 ng of the plasmid pBR322 which had been similarly tailed with deoxy(G) residues at the PstI site. The annealed mixture was then transformed into E. coli K12 strain 294. Approximately 1,500 transformants were obtained.

Since the cDNA priming reaction had been done using a synthetic fragment that hybridized 13 base pairs from the N-terminal of clone pPA25E10, no convenient restriction fragment was available in this 29 base pair region (which includes the 16-mer sequence) for screening the cDNA clones. Therefore, it was necessary to isolate a human tissue plasminogen activator genomic clone in order to identify any primer extended cDNA clones containing N-terminal tissue plasminogen activator coding sequences.

The first step in this process involved establishing the fact that only a single homologous tissue plasminogen activator gene is

0242L

present in human genomic DNA. To determine this, a Southern hybridization was performed. In this procedure, 5 µg of high molecular weight human lymphocyte DNA was digested to completion with various restriction endonucleases, electrophoresed on 1.0 percent agarose gels and blotted to a nitrocellulose filter. A $^{32}$P-labelled DNA probe was prepared from the 5' end of the cDNA insert of the cDNA clone pPA25E10 (a 230 bp HpaII-RsaI fragment) and hybridized with the nitrocellulose filter. 35 x 10$^6$ counts per minute of the probe were hybridized for 40 hours and then washed (Fritsch et al., Cell 19:959, 1980). Two endonuclease digestion patterns provide only a single hybridizing DNA fragment: BglII (5.7 Kbp) and PvuII (4.2 Kbp). Two hybridizing DNA fragments were observed with HincII (5.1 Kbp and 4.3 Kbp). Taken together, these data suggest the presence of only a single tissue plasminogen activator gene in the human genome, and that this gene contains at least one intervening sequence.

The strategy used to identify λ phage recombinants carrying tissue plasminogen activator genes consisted in detecting nucleotide homology with a radioactive probe prepared from the tissue plasminogen activator clone pPA25E10. One million recombinant λ phage were plated out on DP 50 Sup F at a density of 10,000 pfu/15 cm plate, and nitrocellulose filter replicas were prepared for each plate by the method of Benton and Davis (Science 196:180, 1977). A $^{32}$P-labelled DNA probe was prepared by standard procedures from a 230 base pair HpaII-RsaI fragment located 34 base pairs from the 5' end of the clone p25E10. Each nitrocellulose filter was prehybridized at 42° for 2 hours in 50 mM sodium phosphate (pH 6.5), 5X SSC (77), .05 mg/ml sonicated salmon sperm DNA, 5X Denhardt's solution, 50 percent formamide and then hybridized with 50 x 10$^6$ counts per minute of the labelled probe in the same solution containing 10 percent sodium dextran sulfate. After an overnight incubation at 42°C, the filters were washed 4 times at 50° in 0.2X SSC, 0.1 percent SDS for 30 minutes, once in 2 x SSC at room temperature and then exposed to Kodak XR-5 X-ray film with Dupont Cronex intensifying screens overnight. A total of 19 clones were obtained which hybridized with the probe. Phage DNA was prepared as previously described from 6 recombinants. λ Clone C was selected for preparation of a PvuII fragment for colony screening. 30 μg of DNA was digested with PvuII for 1 hour at 37°, and electrophoresed on 1.0 percent agarose gels. A 4.2 Kilobase pair fragment previously shown to contain tissue plasminogen activator sequences was electroeluted and purified. A $^{32}$P labelled probe was prepared by standard procedures for colony hybridizations as described infra.

The colonies were transferred from plates and grown on nitrocellulose filters and the DNA from each colony fixed to the filter by the Grunstein-Hogness procedure. A $^{32}$P-labelled probe was made by calf-thymus priming (Taylor et al., Biochem. Biophy. Acta 442:324, 1976) a 4.2 kilobase pair PvuII fragment from an isolated tissue plasminogen activator λ genomic clone. Filters containing the 1,500 transformants were hybridized with 112 x 10$^6$ cpm of $^{32}$P-genomic PvuII fragment. Hybridization was for 16 hours using conditions described by Fritsch et al. Filters were extensively washed and then exposed to Kodak XR-5 X-ray film with Dupont Lightning-Plus intensifying screens for 16-48 hours. Eighteen colonies clearly hybridized with the genomic probe. Plasmid DNA was isolated from each of these colonies and was bound to nitrocellulose filters and hybridized with the $^{32}$P-labelled synthetic oligonucleotide (16-mer) used for the original priming reaction. Of the 18 clones, seven hybridized with the kinased 16-mer. Upon sequence analysis after subcloning fragments into the m13 vector mp$^7$ (73), one clone (pPA17) was shown to contain the correct 5' N terminal region of tissue plasminogen activator, a signal leader sequence and an 84 bp 5' untranslated region. From the two clones pPA25E10 and pPA17 the complete nucleotide sequence and restriction pattern of a full length tissue plasminogen activator clone were determined. Three fragments from overlapping tPA plasmids, pPA25E10, and pPA17, are pΔRIPA are prepared as follows: Plasmid pPA17 is digested with DdeI, filled in using Klenow DNA polymerase 1, and subcut with PstI; the approximately 190 bp fragment containing 5' terminal tPA sequence thus generated was isolated. The second tPA fragment is obtained by digesting PΔR1PA° with PstI and NarI and isolating the 320 bp fragment. The third tPA fragment is obtained by digesting pPA25E10 with NarI and BglII and isolating the 1645 bp fragment which contains, in addition to much of the tPA coding region, some 3' non-translated sequences.

The HBV surface antigen expressing plasmid p342E (also referred to as pHBs348-E) has been described by Levinson et al., Patent Application Serial No. 326,980, (EP 0073656), which is hereby incorporated by reference, and is similar to pCVSVEHBV except that the promoter is a 342 bp PvuII-HindIII fragment encompassing the SV40 origin. pE342 is modified by digesting with trace amounts of EcoRI, filling in the cleaved site using Klenow DNA polymerase I, and ligating the plasmid back together, thus removing the EcoRI site preceding the SV40 origin in pE342. This plasmid, designated pE342ΔR1, is digested with EcoRI, filled in using Klenow DNA polymerase I, and subcut with BamHI. After electrophoresing on an acrylamide gel, the approximately 3500 bp fragment is electroeluted, phenol-chloroformed, and ethanoled as above.

Plasmid pE342 which expresses HBV surface antigen (also referred to as pHBs348-E) has been described by Levinson et al, Patent Application Serial No. 326,980, filed December 3, 1981, which is incorporated herein by reference (EPO Publication No. 0073656).

The p342E 3500 bp vector, and above described tPA fragments comprising approximately 2160 bp were ligated together using standard techniques. A plasmid containing the three tPA encoding fragments in the proper orientation was isolated, characterized, and designated pE342-t-PA. This plasmid was digested with SacII and treated with bacterial alkaline phosphatase (BRL). To provide the DHFR sequence (along with control sequences for its expression) an approximately 1700 bp fragment was generated by SacII digestion of pEHER. This fragment was ligated into the pE342-t-PA plasmid to create pEdPAER400, a plasmid which is analogous to pEHER except that the HBsAg coding region has been replaced by the cDNA sequences from t-PA.

pETPAER400 (pETPER) was transfected into both dhfr⁻ CHO-DUX Bll cells and DHFR⁺ CHO-K1 (ATCC CCL61) cells. Transformed dhfr⁻ cells were selected by growth in glycine, hypoxanthine and thymidine deficient medium. Transformed DHFR⁺ cells were selected by growth in $\geq$100 nM MTX. Colonies which arose on the appropriate selection medium were isolated using cloning rings and propagated in the same medium to several generations.

For amplification cells from the colonies are split into media containing $5 \times 10^4$, $10^5$, $2.5 \times 10^5$, $5 \times 10^5$, and $10^6$ nM MTX and passaged several times. Cells are plated at very low ($10^2$-$10^3$ cells/plate) cell densities in 10 cm dishes and the resulting colonies are isolated.

In a manner analogous to that used in the construction of pETPER, a plasmid containing the DNA sequence encoding wild type DHFR, pETPFR, was constructed. The construction was as described

0242L

above but using    in place of plasmid pEHER as a source for the DHFR protein gene sequence, the plasmid pE342.HBV.E400.D22. The plasmid pE342.HBV.E400.D22 is the same as pEHER except for a single base pair difference between wild type and mutant DHFR. It can be constructed analogously to pEHER, using pFD11 in place of pFR400. Thus the resulting plasmid pETPFR is analogous in every way to pETPER except that the DNA sequence encoding for wild type DHFR is substituted for that of the mutant.

pETPAER400 (pETPER) was transfected into both dhfr- and DHFR+ CHO cells as described above. After selection, either with glycine, hypoxanthine and thymidine deficient medium for dhfr- cells, or with MTX for the CHO-K1 cells, colonies were isolated using cloning rings and propagated in the selective medium to several generations. For amplification cells from the colonies will be split into $5 \times 10^4$, $10^5$, $2.5 \times 10^5$, $5 \times 10^5$, and $10^6$ nM MTX and passaged several times. Cells are plated at very low ($10^2$-$10^3$ cells/plate) cell densities in 10 cm dishes and the resulting colonies are isolated as usual.

Expression of tPA in the transfected amplified colonies is assayed as follows. Media from pETPAER400 transfected cells are added to an agarose plate containing fibrin and plasminogen. Samples having tPA activity rapidly clear a zone around the well in the otherwise opaque gel Goeddel et al., supra; Granelli-Piperno and Reich, J. Exp. Med. 148:223 (1978).

Coamplification of DHFR and tPA sequences is assayed by isolating DNA from confluent monolayers of amplified colonies as follows: Confluent monolayers in 150 mm plates are washed with 50 ml sterile PBS and lysed by the addition of 5 mls of 0.1 percent SDS, 0.4 M CaCl$_2$, 0.1 M EDTA, pH 8. After 5-10 minutes, the mixture is removed, phenol extracted, chloroform extracted, and ethanol precipitated. The DNA is resuspended in 1 ml (per 150 mm plate) 10 mM Tris pH 8, 1 mM EDTA (TE), RNase added to 0.1 mg/ml, and the solution incubated 30 minutes at 37°. SDS is then added to 0.1 percent and pronase (Sigma) is added to 0.5 mg/ml. After 3-16 hours incubation at 37°, the solution is again phenol extracted, chloroform extracted, and ethanol precipitated as usual. The DNA pellet is resuspended in 0.5 ml water and digested with restriction enzymes as per the standard protocol. Approximately 5-10 µg of digested DNA is electrophoresed in an agarose gel [1 percent agarose in Tris-acetate buffer (40 mM Tris, 1 mM EDTA, made to pH 8.2 with acetic acid) (Crouse et al., J. Biol. Chem. 257:7887, 1982). After bromphenol blue dye had migrated 2/3 of the way down the gel, the gel is removed and stained with ethidium bromide. After visualizing the DNA with ultraviolet light, the gel is treated with HCl, NaOH, and NaCl-Tris and transferred to nitrocellulose filters according to the procedure of Southern (J. Mol. Biol. 98:503, 1975). The filters are then hybridized with a nick translated probe made from the 1700 bp SacII fragment of pEHER (prepared and hybridized as described above), or from the 1950 bp BglII fragment of pPA25E210.

0242L

CLAIMS

1.    A nucleotide sequence of the structure substantially as is set forth in Figure 1b hereof.

2.    A nucleotide sequence coding for the amino acid sequence of DHFR 3T6-R400, substantially free of impurities and of intron sequences.

3.    An expression vector which comprises the DNA sequence coding for the amino acid sequence of a DHFR protein with a low binding affinity for MTX operably linked to a DNA sequence capable of effecting expression thereof.

4.    A replicable plasmid capable, in a transformant eukaryotic host cell, of expressing DHFR protein with a low binding affinity for MTX.

5.    The plasmid of claims 3 or 4 wherein the DHFR protein is DHFR 3T6-R400.

6.    The plasmid pFR400.

7.    A eukaryotic cell line transformed with the vector of any one of claims 3 to 6.

8.    An expression vector which comprises a first DNA sequence encoding a DHFR protein with a low binding affinity for MTX and a second DNA sequence encoding a desired heterologous protein, each of said first and second sequences being operably linked to a DNA sequence capable of effecting expression of the respective coding sequence.

9.    The vector of claim 8 wherein the first and second DNA sequences are operably linked to a single DNA sequence capable of effecting expression of both said sequences.

10. The vector of claim 8 or 9 wherein the DHFR protein is DHFR 3T6-R400.

11. The vector of claim 8, 9 or 10 wherein the desired heterologous protein is HBsAg or tPA.

12. The plasmid pCVSVEHBVE400R400 or pETPAER400.

13. A method of transforming eukaryotic cells which method comprises mixing said cells with the expression vector of any one of claims 3 to 6 and 8 to 12.

14. A method of selecting transfected eukaryotic cells which method comprises mixing said cells with the expression vector of any one of claims 3 to 6 and 8 to 12 and growing the cells in a medium containing a concentration of MTX effective in selecting transformed cells.

15. A method of amplifying the DNA sequence encoding a desired heterologous protein in a eukaryotic host cell culture, which method comprises mixing said cells with the vector of any one of claims 8 to 12 and growing the cells in the presence of am amplifying concentration of MTX.

16. A method of increasing the production of a desired heterologous protein in a eukaryotic host cell culture, which method comprises mixing said cells with the vector of any one of claims 8 to 12 and growing the cells in the presence of an amplifying concentration of MTX.

17. A eukaryotic host cell culture transformed with the vector of any one of claims 8 to 12.

18. The method of any one of claims 13, 15 and 16 wherein the host cell culture is CHO K1.

19.    A method of selecting transfected vertebrate cells which method comprises providing a replicable expression vector comprising a DNA sequence encoding for a protein which confers drug resistance operably linked to a DNA sequence capable of effecting expression thereof.

20.    A method of selecting transfected vertebrate cells which method comprises providing a replicable expression vector comprising a DNA sequence encoding for desired heterologous protein and a DNA sequence encoding for a protein which confers drug resistance each operably linked to a DNA sequence capable of effecting expression thereof.

21.    A method according to claim 16 which further includes extracting the heterologous protein from the culture.

22.    The method of claim 21 wherein the heterologous protein is HBsAg or tPA.

Fig. 1a.

PstI  Fnu4HI  TaqI  HinfI  TaqI  HinfI  BglII  PstI

ml3

ml3

100 bp

# Fig. 1b.

```
               A                                                                    G                                      Fnu4HI
GGGGCGGGGCCTTAGCTGCGCAAGTGGTACACAGCTCAGGGCTGCGATTTCGCGCCAAACTTCACGGCAATCCTAGCGTGAAGGCTGGTAGGATTTTATCCCCGCTGCCATC    ATG
                                                                                                                      ┌────┐
       TaqI                                            10                                         20                   │leu │          30
                                                                                                                      │ T  │
       val arg pro leu asn cys ile val ala val ser gln asn met gly ile gly lys asn gly asp arg pro trp pro pro leu arg asn glu
       GTT CGA CCA TTG AAC TGC ATC GTC GCC GTG TCC CAA AAT ATG GGG ATT GGC AAG AAC GGA GAC CGA CCC TGG CCT CCG CTC AGG AAC GAG

                                            40                                   HinfI     50                                60
       phe lys tyr phe gln arg met thr thr thr ser ser val glu gly lys gln asn leu val ile met gly arg lys thr trp phe ser ile
       TTC AAG TAC TTC CAA AGA ATG ACC ACA ACC TCT TCA GTG GAA GGT AAA CAG AAT CTG GTG ATT ATG GGT AGG AAA ACC TGG TTC TCC ATT

               TaqI                          70                                           80                                90
       pro glu lys asn arg pro leu lys asp arg ile asn ile val leu ser arg glu leu lys glu pro pro arg gly ala his phe leu ala
       CCT GAG AAG AAT CGA CCT TTA AAG GAC AGA ATT AAT ATA GTT CTC AGT AGA GAA CTC AAA GAA CCA CCA CGA GGA GCT CAT TTT CTT GCC

                                            100                                          110                               120
       lys ser leu asp asp ala leu arg leu ile glu gln pro glu leu ala ser lys val asp met val trp ile val gly gly ser ser val
       AAA AGT TTG GAT GAT GCC TTA AGA CTT ATT GAA CAA CCG GAA TTG GCA AGT AAA GTA GAC ATG GTT TGG ATA GTC GGA GGC AGT TCT GTT

                                            130                                          140                               150
       tyr gln glu ala met asn gln pro gly his leu arg leu phe val thr arg ile met gln glu phe glu ser asp thr phe phe pro glu
       TAC CAG GAA GCC ATG AAT CAA CCA GGC CAC CTC AGA CTC TTT GTG ACA AGG ATC ATG CAG GAA TTT GAA AGT GAC ACG TTT TTC CCA GAA

                                            160                                          170                               180
       ile asp leu gly lys tyr lys leu leu pro glu tyr pro gly val leu ser glu val gln glu glu lys gly ile lys tyr lys phe glu
       ATT GAT TTG GGG AAA TAT AAA CTT CTC CCA GAA TAC CCA GGC GTC CTC TCT GAG GTC CAG GAG GAA AAA GGC ATC AAG TAT AAG TTT GAA

                       186
       val tyr glu lys lys asp OC                                                                                    BglII
       GTC TAC GAG AAG AAA GAC TAA CAGGAAGATGCTTTCAAGTTCTCTGCTCCCCTCCTAAAGCTATGCATTTTTATAAGACCATGGGACTTTTGCTGGCTTTAGATCTATGAGTAA

                                       G  CC CCC    C C                                                                            (T)
       TTATTTCTTTAGGGAGGGGTAGTTGGAAGAATTGTTTGTTTTGTGATCTTGGGGATGGAACCTAAGACCCAGTGCGTGCTGAGCAAATGCTATACTGCTGAGCCACCCCAACCCTAGCCC

                               T  C   CT                       T C-  TT        TC    -    -        (T)          (GA)
       CTATATAATTCTAAACAATATGTTGTCATTTCCCAGTAATCTAACAAGGTATAGTAAAAGTGCCTTAAGAAATGTCACTTGCTATAAAGGTCTCAGTGCCCCTCCCATGAGACCTCAAGT

       CCCCCCCCCCCCCCCCCCCCCCCCCCCCCCCCCCCCCCCCCCCCCC
```

# Fig.2.

GATC        GATC

G→

T→

MUTANT        WILD TYPE

Fig.3.

Fig.4.

Percent of Control — MTX (nM)

dhfr⁻/pFDII   CHO-KI   dhfr⁻/pFR400

Fig. 5.

Fig.6.